(19)

Europäisches
Patentamt
European
Patent Office
Office européen
des brevets

(11) **EP 3 587 586 A1**

(12) **EUROPEAN PATENT APPLICATION**

(43) Date of publication:
**01.01.2020 Bulletin 2020/01**

(21) Application number: **18179371.2**

(22) Date of filing: **22.06.2018**

(51) Int Cl.:
*C12Q 1/68* (2018.01)    *G06F 19/18* (2011.01)
*C12Q 1/6869* (2018.01)    *G06F 19/22* (2011.01)
*G06F 19/12* (2011.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Julius-Maximilians-Universität Würzburg**
**97070 Würzburg (DE)**

(72) Inventors:
• **Erhard, Florian**
  **97072 Würzburg (DE)**
• **Dölken, Lars**
  **97070 Würzburg (DE)**

(74) Representative: **Lucke, Andreas**
  **Boehmert & Boehmert**
  **Anwaltspartnerschaft mbB**
  **Pettenkoferstrasse 22**
  **80336 München (DE)**

(54) **METHOD FOR STATISTICALLY DETERMINING A QUANTIFICATION OF OLD AND NEW RNA**

(57)    The present invention provides a method for determining a quantification of old and new RNA for a gene, the method comprising:
- obtaining reads from RNA fragments of a sample, wherein the RNA fragments comprise nucleotide substitutions caused by a metabolic marker,
- determining statistics of nucleotide mismatches in the reads,
- determining, based on the statistics of nucleotide mismatches, an error rate indicating a rate of nucleotide mismatches in old RNA and a conversion rate indicating a rate of nucleotide mismatches in new RNA, and
- determining the quantification of old and newly synthesized RNA for the gene based on the error rate, the conversion rate and the statistics for the gene.

FIG. 7

**Description**

**BACKGROUND**

Gene expression is the process by which genetic information is converted and made usable for the cell. This includes the transcription of DNA into RNA followed by its translation into proteins. Gene expression is quantitative (i.e. it is important how much RNA is present), regulated (i.e. there are mechanisms that can control the strength of transcription and translation gene specifically) and highly dynamic (genes are expressed to different degrees in different cells and at different times and conditions).

RNA is constantly degraded (with an RNA-specific degradation rate) and new RNA is constantly produced (with a specific transcription rate) in order to maintain steady state levels. An important indicator of each mRNA is its turn-over rate, which determines the time it takes until RNA levels establish steady state from any non-steady state (e.g. when transcription and/or degradation rates have changed). mRNA half-lives range from a few minutes to more than 24 hours (how long does it take to reach halfway to the new steady state).

Total amounts of all expressed RNAs of all genes can be determined with the help of so-called RNA-seq analyses. All mRNAs in a sample are fragmented and transcribed into cDNA. Millions of these fragments are then sequenced, for example using Illumina sequencing. The number of sequencing "reads" received for a specific mRNA can then be used as a measure of its expression strength. For each of the millions of reads, it is first determined where in the genome (or in the totality of the mRNA sequences) its sequence occurs ("read mapping"). Often two experimental conditions are compared (e.g. virus-infected sample against control sample). When conditions involve fast processes (e.g. two hours after virus infection), changes in the transcription or degradation rate are only reflected to a small extent in total RNA levels. RNAs with different turn-over rates (especially mRNAs with medium and long half-lives) are affected to varying degrees. This can introduce severe bias into any analyses. To avoid this, in the method of 4sU-tagging by Dölken et al., 2008, the nucleoside analogue 4-thiouridine (4sU) is added to the cell culture medium, rapidly absorbed by the cells and incorporated into newly-transcribed RNA. This RNA is thus marked as "new" and can then be biochemically separated from unlabeled (i.e. "old") RNA. Total RNA, new RNA and old RNA can then be analysed separately. This allows changes in RNA synthesis but also in RNA degradation (via the ratio of "new" to "old" to "total" RNA) to be determined. Standard tools for RNA-seq are sufficient for most of the analyses.

The goal of SLAM-seq (Herzog et al., 2017) is to achieve the separation of old and new RNA without complex biochemistry. For this purpose, the RNA isolated from the cells is treated with iodoacetamite (IAA) before preparing the samples for sequencing, which reacts with the nucleoside analogue 4-thio-uridine (4sU) and causes 4sU to be read in cDNA synthesis not as uracil (U) but as cytosine (C). As alternatives to IAA, other chemical agents were used for conversion (TimeLapse-seq and TUC-seq) (Riml et al., 2017; Schofield et al., 2018). The separation can therefore take place after sequencing by looking at each read and considering positions in the read where U was in the mRNA sequence but C was sequenced (or, if mapped to the genome: positions which are a thymine (T) in the genome but were sequenced as C). Many reads with such T to C mismatches indicate many new mRNAs for a gene. It is therefore critical in the analysis of such data to decide correctly whether mismatches were caused by conversion of 4sU or for other reasons (sequencing errors, SNPs,...).

For this purpose, the SLAM-dunk analysis program requires additional samples taken without 4sU input (no4sU samples). After read mapping to the genome sequence using a program specifically adapted for SLAM-seq (Next-Gen-Map, which can be configured not to penalize T to C mismatches), all positions covered at least ten times, which in more than 80% of the cases have a mismatch, are first filtered as SNPs. Reads for each gene and sample are counted and compared between samples using standard approaches (normalization to counts per million mapped reads, CPM). For each gene, the difference of these normalized values is calculated from the considered 4sU sample and a no4sU sample. This number is then divided by the T-coverage (how many T would have been sequenced without mismatches) of this gene. This number is reported by SLAM-dunk as the conversion rate. Although this number is correlated with the proportion of new to total RNA (the higher the number, the larger the proportion), there is a need for a method that can provide fully quantitative analyses of new and old RNA.

**SUMMARY OF THE INVENTION**

A first aspect of the present invention provides a method for determining a quantification of old and new RNA for a gene, the method comprising:

- obtaining reads from RNA fragments of a sample, wherein the RNA fragments comprise nucleotide substitutions caused by a metabolic marker,
- determining statistics of nucleotide mismatches in the reads,
- determining, based on the statistics of nucleotide mismatches, an error rate indicating a rate of nucleotide mismatches in old RNA and a conversion rate indicating a rate of nucleotide mismatches in new RNA, and
- determining the quantification of old and newly synthesized RNA for the gene based on the error rate, the conversion rate and the statistics for the gene.

The quantification may be a ratio of new over total RNA or may be a probability distribution of a ratio of new over old RNA. New RNA may refer to the RNA synthesized in presence of the metabolic marker whereas old RNA may refer to the not yet degraded RNA synthesized prior to adding the metabolic marker.

The sample is preferably a single sample and may be taken from a single cell.

Nucleotide mismatches can be determined e.g. by comparison with a reference genome of the one or more cells.

The method can be performed equivalently for a plurality of genes. The one or more cells may be a single cell.

The statistics preferably comprise a number n of nucleotides that may be exchanged caused by the metabolic marker and a number k of observed nucleotide mismatches. "Statistics for the gene" refers to that part of the statistics of the reads that covers the gene.

The probability distribution of the ratio of newly synthesized RNA is preferably obtained using Bayesian statistics. Thus, it may also be referred to as posterior probability distribution or just "posterior".

The method of the first aspect has the advantage that based on only one sample the error rate, the metabolic conversion rate and the ratio of newly synthesized RNA can be determined.

In a first implementation of the method of the first aspect, the metabolic marker is 4sU and/or the nucleotide mismatches are T to C mismatches.

In a further implementation of the method of the first aspect, the method further comprises further a preprocessing step of removing from the statistics nucleotide substitutions which, due to their high number, cannot be explained as conversions by the metabolic marker.

In a further implementation of the method of the first aspect, determining the error rate comprises using a linear regression model that has been trained with reads of cells that have not been metabolically labelled to predict the error rate based on error rates of mismatches that do not correspond to a metabolically caused mismatch.

Advantages of embodiments of the method of the first aspect include that the amount of new RNA be determined, the actual new/old ratio (or the specific RNA half-life) or an estimation of the statistical error, which is important for such small numbers, may be determined.

In a further implementation of the method of the first aspect, determining the conversion rate comprises:

- determining a number k of nucleotide mismatches for which less than a predetermined ratio of observed reads is expected to originate from RNA that is not metabolically labelled, and
- determining the conversion rate based on statistics where k or fewer mismatches are excluded.

The predetermined ratio may have a value between 0,1 and 0,001, in particular between 0,03 and 0,003, for example 0,01.

In a further implementation of the method of the first aspect, the reads are acquired using paired-end-sequencing and wherein the method further comprises determining a double-stranded error rate indicating a rate of nucleotide mismatches in double-sequenced parts of read pairs of old RNA and/or a double-stranded conversion rate indicating a rate of nucleotide mismatches in double-sequenced parts of read pairs of new RNA.

Paired-end sequencing provides two significant advantages over single-end reads: First, error rates can be estimated from the other read, since for example 4sU converted to cytosine results in a T to C mismatch in the first read, and in an A to G mismatch in the second read. Second, especially if RNA is strongly fragmented, read pairs overlap. All nucleotides in the overlapping part are sequenced twice, making the differentiation of true conversions from sequencing errors much easier: The probability for two independent sequencing errors of the same nucleotide is negligible small and this cause of mismatches can therefore be excluded.

In a further implementation of the method of the first aspect, the method further comprises determining the RNA half-life $\lambda$ of the gene according to

$$\lambda = \frac{\log(2)}{-\frac{1}{t}\log(1 - \pi)}$$

wherein t is the effective labeling time of the metabolic marker, in particular of 4sU, and $\pi$ is the ratio of new to total RNA.

In a further implementation of the method of the first aspect, the method further comprises

- determining a probability distribution of a ratio of new-to-total RNA for a plurality of samples,

- for each of the plurality of samples, determining parameters of a beta distribution that approximates the probability distribution, and
- determining an estimator of the decay rate or a posterior distribution of the estimator of the decay rate based on the parameters of the plurality of plurality of samples, and/or
- determining an estimator of the RNA half-life or a posterior distribution of the estimator of the RNA half-life based on the parameters of the plurality of plurality of samples.

In a further implementation of the method of the first aspect, determining parameters of a beta distribution that approximate the probability distribution comprises fitting parameters of a beta distribution by numerically minimizing a distance measure between the probability distribution and the beta distribution. For example, the numerically minimizing can be performed by minimizing a distance measure at Newton Cotes supporting points.

In a further implementation of the method of the first aspect, the estimator is determined by numerically maximizing:

$$l(\delta) = \sum_i (\alpha_i - 1) \log(1 - e^{-t_i \delta}) - t_i \beta_i \delta$$

wherein $\delta$ is the decay rate, $\alpha_i$, $\beta_i$ are parameters of an i-th sample, and $t_i$ is a labeling time of the metabolic marker in the i-th sample.

In a further implementation of the method of the first aspect, the method further comprises determining, based on the probability distribution of the ratio of newly synthesized RNA of the gene and/or based on the probability distribution of the RNA half-life of the gene, a biological significance of the sample.

When investigating whether a particular treatment has an effect on new synthesis or half-life, a suitable statistical test per gene is usually applied to replicated experiments. Measurement inaccuracies of individual samples can lead to false-negative results (i.e. reduce the statistical power of the test). The posterior distributions (those of new/total ratios, but also of RNA half-lives) provide an internal quality measure per sample and gene. Thus, inaccurately measured samples per gene can be excluded from the statistical test on the basis of a determined biological significance of the sample, in particular based on the posterior distributions. This substantially increases the power of the statistical test.

In a further implementation of the method of the first aspect, the method further comprises rejecting or accepting a determined value of the sample based on the determined biological significance.

In a further implementation of the method of the first aspect, instead of new RNA old RNA is labeled by the metabolic marker. For example, labelling can be performed for a long duration such that virtually all RNAs are labelled. Then the marker can be washed out such that new RNA does not comprise 4sU.

A second aspect of the present invention provides a device for determining a quantification of old and new RNA for a gene, the device comprising:

- an obtaining unit for obtaining reads from RNA fragments of a sample, wherein the RNA fragments comprise nucleotide substitutions caused by a metabolic marker,
- a statistics unit for determining statistics of nucleotide mismatches in the reads,
- a sample analysis unit for determining, based on the statistics of nucleotide mismatches, an error rate indicating a rate of nucleotide mismatches in old RNA and a conversion rate indicating a rate of nucleotide mismatches in new RNA, and
- a gene analysis unit for determining the quantification of old and newly synthesized RNA for the gene based on the error rate, the conversion rate and the statistics for the gene.

The device of the second aspect may be configured to carry out the method of the first aspect or one of its implementations.

A third aspect of the present invention provides a computer-readable storage medium storing program code, the program code comprising instructions that when executed by a processor carry out the method of the first aspect or one of its implementations.

## BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description merely relate to embodiments of the present invention, modifications on these embodiments are possible without departing from the scope of the present invention as defined in the claims.

FIG. 1    is a flow chart of a method in accordance with an embodiment of the present invention,
FIG. 2    is a table and diagram of sufficient statistics as used in the embodiment of the invention,
FIG. 3    is a diagram illustrating a binomial distribution of T to C mismatches in the example of FIG. 2,
FIG. 4    is a diagram illustrating the distribution of old and new reads in the example of FIG. 2,
FIG. 5    shows two diagrams illustrating the statistics for all old or all new reads,
FIG. 6    shows two diagrams illustrating statistics for two genes with 100 reads,
FIG. 7    is a schematic illustration of aspects of the method of FIG. 1,
FIG. 8    shows diagrams illustrating the validation by simulation,
FIG. 9    shows diagrams illustrating the influence of read mapping,
FIG. 10    shows an evaluation of mESC data,
FIG. 11    shows diagrams illustrating the RNA half-life,
FIG. 12    illustrates Pearson's correlation coefficients for RNA half-lifes, and
FIG. 13    shows a differential analysis of results of the method.

## DETAILED DESCRIPTION OF EMBODIMENTS

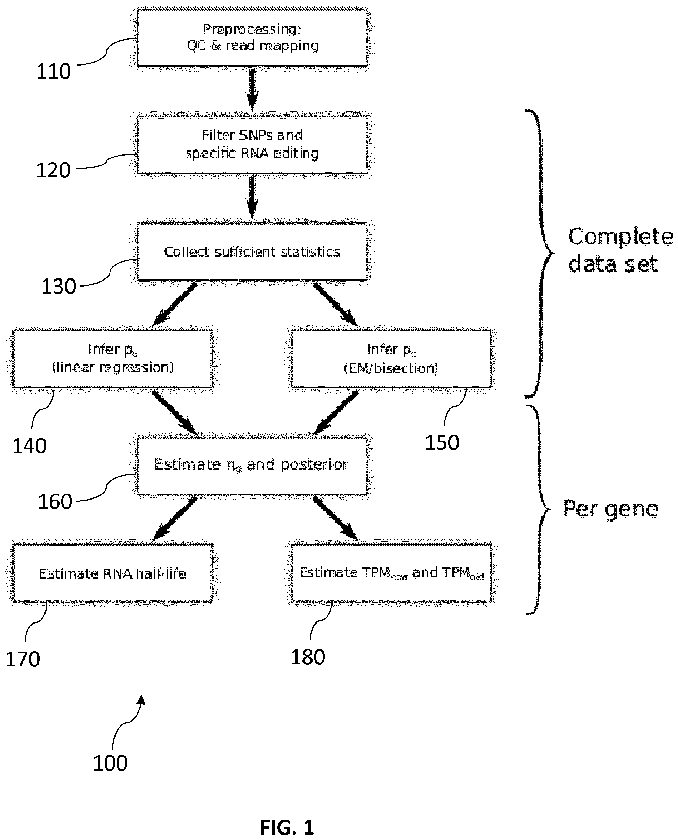

FIG. 1 is a flow chart of a method 100 for determining a quantification of old and new RNA for a gene.

In a first step 110, preprocessing is performed. This may involve quality control and mapping of reads to the genome. Mapping of the reads from sequencing may be performed with STAR.

In contrast to Next-Gen-Map, STAR is a widely used and very fast software, but not directly designed for T after C mismatches. However, with the help of simulation experiments we were able to show that the special position of T to C in Next-Gen-Map has no effect on the results obtained.

In a second step 120, SNPs and specific RNA editing is filtered. In particular, positions may be filtered that indicate SNPs (or generally: positions with frequent T to C mismatches that are not due to 4sU conversion). The method may filter all positions where a statistical test rejects the null hypothesis that the number of observed mismatches is not greater than expected at an assumed maximum 4sU incorporation rate (e.g.: 10%, or a value between 1% and 30%, preferably between 2% and 15%). This means that if the coverage is sufficiently large, filtering is already performed if more than 10% mismatches are observed, but if the coverage is smaller, the statistical error of 10% is taken into account.

The method may remove further U- to C-substitutions, which cannot be explained by 4sU-incorporation due to their high number. For this purpose, a statistical test may be used.

In a third step 130, sufficient statistics are collected. For an efficient calculation of the algorithm it is beneficial to operate on the minimal sufficient statistics and not to use all reads for each iteration of the algorithm. To this end, each read may be first reduced to the number pair (d,n), where n is the number of covered genomic thymines (T), and d is the number of T-to-C mismatches. The number of reads for all possible number pairs (d,n) in the entire sample can be counted in a table A with N (=maximal n) columns and D (=maximal d) rows. Such a table can also be determined per gene. These tables represent the minimal sufficient statistics, in the following just referred to as the statistics.

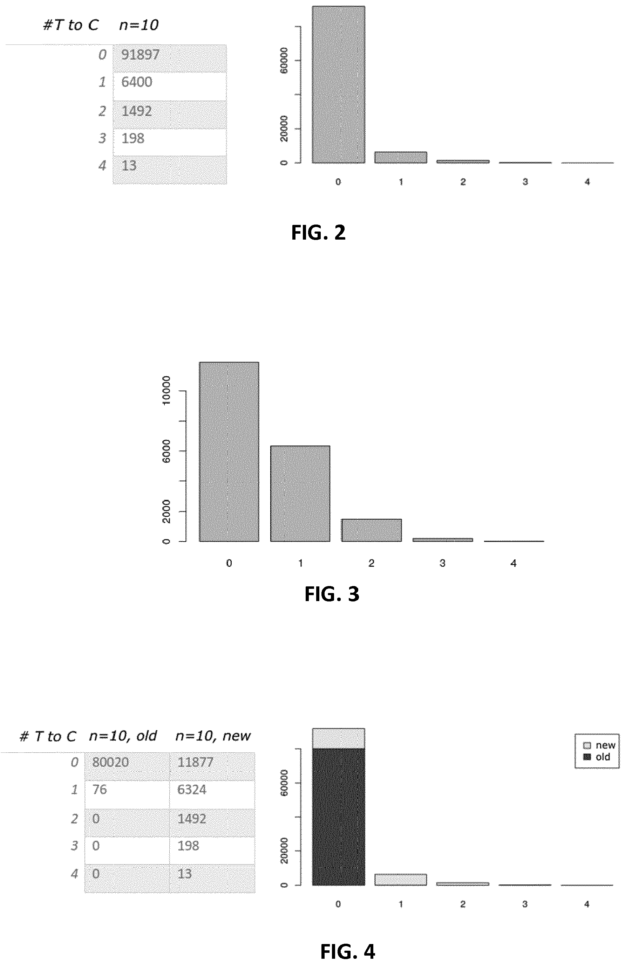

For a very simplified example, we assume that a total of 100 000 reads have been sequenced, all covering exactly 10 genomic Ts. The table contains a column with values as indicated in FIG. 2. The diagram indicates the number of sequenced reads where a C was sequenced at 0, 1, 2, 3 or 4 positions instead of a T.

In a fourth step 140 and a fifth step 150, the probability of sequencing a single genomic T as a C in an old RNA (error rate, $p_e$) and the probability of sequencing a single genomic T as a C in a new RNA (conversion rate, $p_c$) are determined. $p_e$ primarily contains sequencing errors, but also promiscuous (i.e. non-specific and very rare) RNA editing or transcription and PCR errors, and all other processes that lead to the fact that what is encoded in the genome as thymine is nevertheless sequenced as C without the presence of 4sU. In contrast, $p_c$ also contains the incorporation and conversion rate of 4sU.

In the fourth step 140, the error rate $p_e$ could in principle be determined from a no4sU sample that has not been labelled with 4sU. However, these values $p_e$ differ greatly from no4sU sample to no4sU sample (up to a factor of two), and thus the $p_e$ to be determined for the actually interesting 4sU sample will be somewhere within this range of possible values. However, if the estimated $p_e$ is significantly different from the actual $p_e$, the estimated new-total ratio for all genes is strongly biased. Based on the available data, however, we have observed that in no4sU samples the individual mismatch rates (i.e. T to A, T to G,...) are strongly correlated. We could show that the T to C error rate in no4sU samples can be predicted very precisely using linear regression. This regression model is learned once (per type of sequencer) using no4sU samples, and can then be used to estimate $p_e$ very precisely for all future 4sU samples without requiring new no4sU samples.

In the fifth step 150, the conversion rate $p_c$ is determined. In principle, $p_c$ could be determined directly from many reads for which it is known that they originate from new RNA. In this case one could simply count mismatches as for $p_e$ in no4sU samples.

However, a preferable implementation of the fifth step is as follows: In order to lead to the actual procedure, the statistical model on which this counting is actually based is described here: As illustrated in FIG. 3, the number of T after C mismatches per read follows a binomial distribution with unknown parameter $p_e$.

The error rate $p_e$ can now be estimated using the maximum likelihood method, which maximizes the likelihood function of the binomial distribution with respect to the observed data (the bar chart in FIG. 3). For the binomial distribution, this maximum corresponds exactly to the above-mentioned counting method.

However, the observed data consist of a mixture of old and new reads. Since $p_e$(which determines a binomial distribution for the old reads) has been determined already in the fourth step (or at least an upper bound of it is available), a minimum number of mismatches can be determined where one can be sure that only a few (or none) reads are old. In the example of FIG. 4 there are no old reads with at least 2 mismatches. We can therefore estimate $p_c$ with statistical procedures that can handle missing data: We assume that we have not observed the bars for 0 and 1, and only use the rest of the data (number of reads with 2 or more conversions) for the statistical inference.

A maximum likelihood estimator for such data can be found using the Expectation Maximization (EM) method, an iterative method that guarantees that a better solution is found after each step. Convergence can be drastically accelerated with the help of bisection: We know that the parameter we are looking for is between 0 and 1. An iteration of the EM algorithm starting at the currently estimated value p (initially p=0.5) for $p_e$ tells us whether the actual maximum is smaller or greater than p. Assuming it is smaller, we know we need to keep searching in the 0 to 0.5 range. In this case we set the current estimate to 0.25 and determine the further search direction with another EM iteration. This halves the search space in each step, which results in a drastic increase in efficiency.

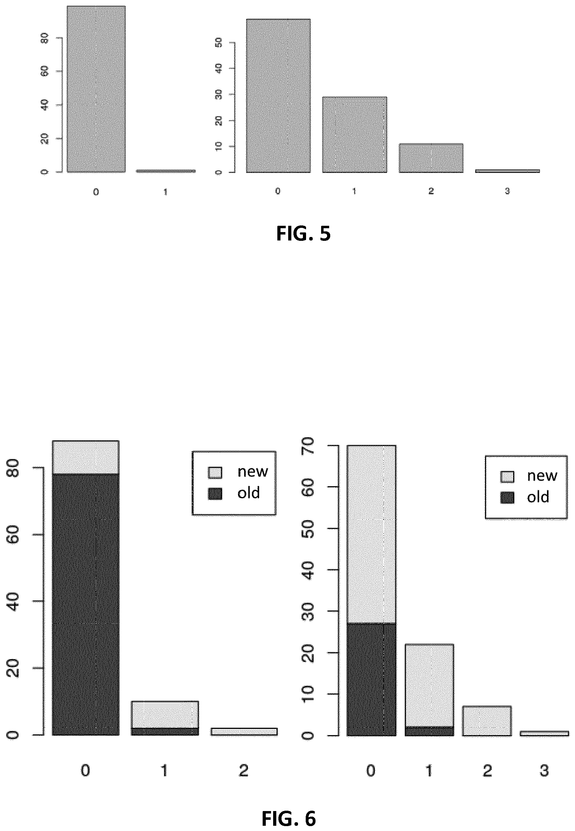

In a sixth step 160, the new to old ratio (or new to total ratio) of RNA is estimated. As introduced above, the observed data can be modeled using a mixture of two binomial distributions. This also applies to data observed for a single gene. Assuming the gene was no longer transcribed (i.e. all reads are old), then the data is binomial-distributed with parameter $p_e$. If the entire old RNA has been degraded (i.e. all reads are new), then the data is binomial-distributed with parameter $p_c$. These two extreme cases are illustrated in FIG. 5.

These limiting cases naturally occur for the fewest genes, and therefore the data usually observed for a gene are a mixture of these two binomial distributions, where the mixing ratio is exactly the new-total ratio.

FIG. 6 illustrates data of two genes with 100 reads each, wherein on the left 20% new RNA have been assumed and on the right 70% new RNA have been assumed.

With the help of Bayesian methods, the posterior probability distribution of the new-total ratio (i.e. which range is the actual value with which certainty) can now be estimated. This posterior probability distribution expresses how likely a mixing ratio is for the observed data if the two limiting cases are binomial distributions with $p_e$ and $p_c$ respectively. It is wide when few reads have been observed for a gene and the difference between $p_e$ and $p_c$ is small.

The posterior probability distribution cannot be analytically determined for the model and is therefore calculated numerically using the Newton-Cotes method. For simultaneously high resolution and efficient computation, the method first determines the relevant range between 0 and 1, in which most of the probability mass of this distribution lies (using a bisection method in which the two points at which the posterior probability distribution is less than 1% of the maximum are searched for).

For a simple representation of the entire posterior probability distribution, as well as the possibility of analytically calculating further analyses, an approximative beta distribution is also determined by adapting both parameters numerically (Nelder-Mead method) to all Newton-Cotes support points (least squares method).

In an optional further step (not shown in FIG. 1) use is made of paired-end sequencing.

The lengths of reads in Illumina sequencing are limited (depending on the sequencer and chemistry used, e.g. 150bp). This means that only cDNA fragments are usually sequenced. The so-called "Paired-End-Sequencing" makes it possible to sequence all fragments from both sides. If the original fragment is shorter than 2x the read length, there is a part inside the fragment that has been sequenced twice. Most of $p_e$ is caused by sequencing errors (in the range of $10^{-4}$). In addition, sequencing errors occur independently of each other. Thus, the probability of observing two corresponding sequencing errors at the same fragment position in this double-sequenced part is negligible (in the range $10^{-8}$). This can be exploited because read pairs with double-sequenced T-to-C mismatch are much more likely new than those with T-to-C mismatch outside the double-sequenced part. Thus, in addition to $p_e$ and $p_c$, the method also estimates $d_e$ and $d_c$ for paired-end data, which are the probabilities of double-sequenced T-to-C mismatches in old and new RNAs. The inference runs according to the same principles as for single-sequenced parts. This additional information can then be directly integrated into the mixture model, and usually leads to narrower posterior probability distributions and thus more precise estimates.

In an optional seventh step 170, the RNA half-life is estimated. Under the assumption that RNA levels of a gene are in steady state, the new-total ratio can be transformed into a its half-life according to the equation

$$\lambda = \frac{\log(2)}{-\frac{1}{t}\log(1-\pi)}$$

Therein, $\lambda$ is the RNA half-life, $t$ is the effective time in which 4sU was in the cells (marker duration), and $\pi$ is the new-total ratio. Furthermore, the approximate posterior probability distribution (beta distribution) of the new-total ratio can be transformed into a posterior probability distribution of the half-life (substitution method). This indicates in which range the RNA half-life lies with which certainty for a single 4sU sample. If several 4sU samples from the same system are available (e.g. replicates, or different times), this can be used to determine a common posterior probability distribution of the half-life, from which a precise estimate of the half-life can be determined using the Maximum-A-Posteriori method, for example. Different labelling periods are lead to different precise individual half-life estimates (long labelling allows precise estimation of long half-lives, short labeling allows precise estimation of short half-lives). The method of common posterior probabilitydistributions ensures that the respective measurements for each gene are included in the overall estimate with appropriate weighting.

In an optional eighth step 180, expression values of new and old RNA can be estimated. Using standard methods (transcripts per million transcripts, TPM), an expression value is determined from the total number of reads per gene. The expression value of new (or old) RNA of a gene can then be determined by multiplying the TPM value of the total RNA suitable by the new-total ratio. Again, the posterior probability distribution can be transformed.

In the following, optional features of the method of FIG. 1 are described in more detail.

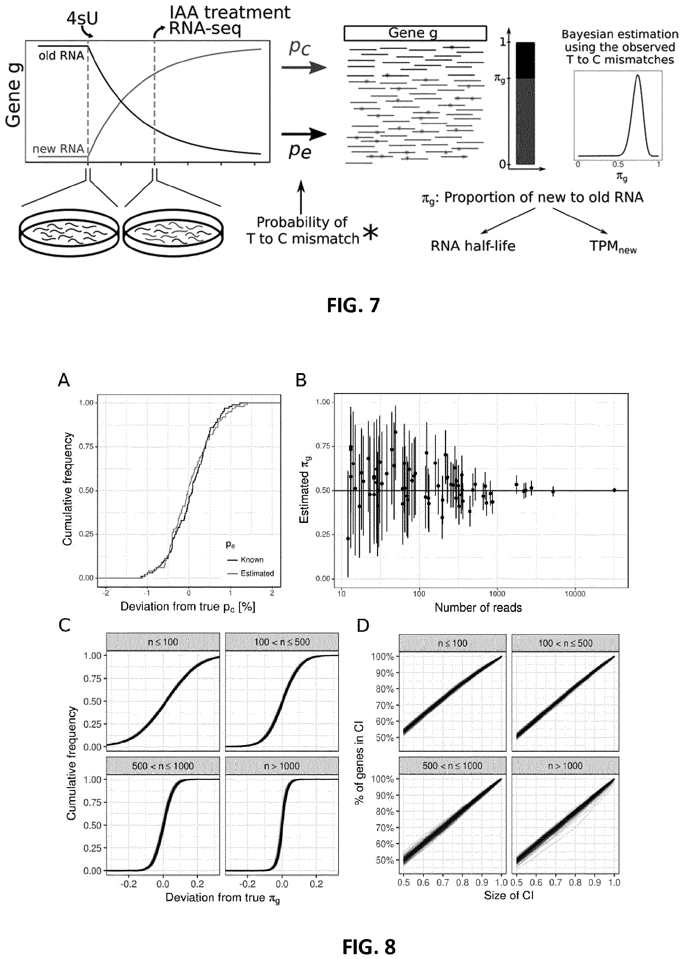

As illustrated in FIG. 7, after a period of labeling with 4-thiouridine (4sU), RNA is extracted from cells, treated with iodoacetamide (IAA) and sequenced. Shown is a theoretical timecourse of the abundances of new and old RNA for a gene g. IAA converts incorporated 4sU into cytosine analogs with an overall rate $p_c$ (including the incorporation rate, conversion rate and error rate), and uridines are sequenced as cytosine with an error rate $p_e$. Based on the observed mismatches from T to C, the proportion of new to total RNA of gene g, $\pi_g$, can be estimated using Bayesian inference. Estimates of $\pi_g$ can be transformed into estimates of the gene's RNA half-life or relative abundance measures.

The approach of the method is based on a binomial mixture model:

$$P(y; p_e, p_c, n, \pi_g) \;=\; (1-\pi_g)B(y; n, p_e) + \pi_g B(y; n, p_c) \quad (1)$$

$$B(k; n, p) \;=\; \binom{n}{k} \cdot p^k (1-p)^{n-k} \qquad (2)$$

The sufficient statistics for this model are the number of observed T to C mismatches (y) for each read mapped to a genomic region containing n thymines within a gene g. If $\pi_g$ is the fraction of newly transcribed RNA among all RNAs of gene g, $p_e$ is the average T to C mismatch rate in unlabeled RNA and $p_c$ is the average mismatch rate in labeled RNA, then observed mismatches for a read are either due to a binomial distribution with success probabilty $p_e$ (with probability $1 - \pi_g$) or a binomial distribution with success probability $p_c$ (with probability $\pi_g$).

Thus, our goal is to estimate all $\pi_g$ using Bayesian inference for observed data $\mathbf{y} = y_1, ..., y_m$ and $\mathbf{n} = n_1, ..., n_m$ (i.e. for each read, how many of the potential T to C mismatches have been observed):

$$f(\pi_g; \mathbf{y}, \mathbf{n}, p_e, p_c) = \frac{\prod_i P(y_i; p_e, p_c, n, \pi_g) \cdot b(\pi_g; \alpha, \beta)}{P(\mathbf{y})} \qquad (3)$$

For the sake of simplicity, we use a beta prior with density function b and hyperparameters $\alpha$ and $\beta$. As we have no prior knowledge on the proportion for each gene, we here use the uninformative uniform prior with $\alpha = \beta = 1$.

The integral $P(\mathbf{y}) = \int_0^1 \prod_i P(y_i; p_e, p_c, n, \pi_g') \cdot b(\pi_g'; \alpha, \beta) d\pi_g'$ is computed numerically. Here, we assume $p_e$ and $p_c$ to be constant throughout a sample. Thus, before solving equation (3) for each gene, we estimate $p_e$ and $p_c$ based on the data from all genes.

The sufficient statistics for parameter estimation are collected in a matrix $A^{(g)}$. Each entry $a_{k,n}^{(g)}$ is the number of reads mapped to a genomic region within gene g containing $n$ thymines with $k$ observed T to C mismatches. We only consider reads consistently mapped to a known transcript (i.e. matching all intron boundaries). Alternatively, for the SLAM-seq experiments (Herzog et al., 2017) where 3' ends of transcripts are sequenced, we only consider reads mapped to the 3' regions defined by SLAM-DUNK (Herzog et al., 2017). In addition we identify and exclude potential SNPs defined as thymines where more than half of the reads covering it show a mismatch.

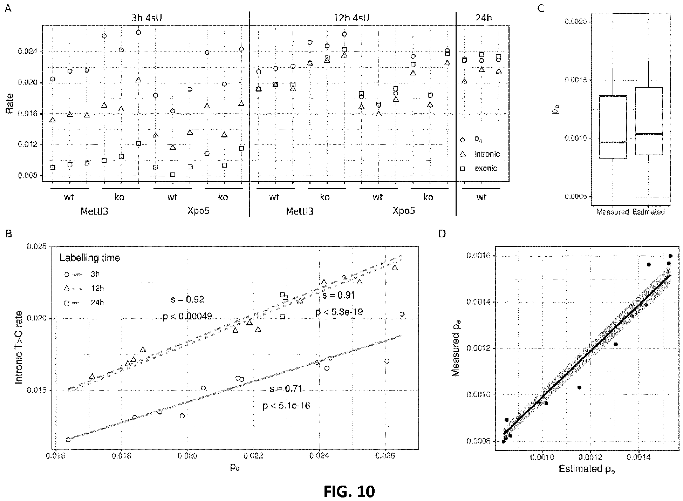

In principle, $p_e$ can be directly estimated from either spike-in RNAs in the same sample, or using an additional sample without 4sU labeling (no4sU sample) by counting T to C mismatches. However, utilizing an additional experiment may lead to a bad estimate for the 4sU sample of interest, as a broad range of $p_e$ values is observed already in available no4sU samples (see FIG. 10C). However, we noticed that the other eleven error rates (one of the nucleotides to any of the other three) are highly correlated to the T to C error rate in the no4sU samples. Thus, we trained a linear regression model to predict the T to C error rate from the other error rates. Manual feature selection revealed that the T to A and T to G error rates alone provided sufficient prediction performance in a leave-one-out cross validation for the data sets used in this study. Consequently, we used the linear regression model based on these two features here (see FIG. 10D), but our implementation can handle any linear regression model or, alternatively, estimates from spike-in RNA.

To estimate $p_c$, we first compute $A = (a_{k,n}) = \Sigma_g A^{(g)}$. Since $y = 0$ is the mode of both component distributions of the binomial mixture model, standard approaches to estimate $p_e$ and $p_c$ based on the expectation maximization (EM) algorithm failed. However, as we can assume that $p_e < p_c$, there is a certain k where only a minor fraction of reads with at least $k$ T to C mismatches originates from the pe component. This k can be computed for each n such that less than 1% of the observed reads with $\geq$ k mismatches is expected to originate from unlabeled RNA. Thus, for each n and k we compute

$$e_{k,n} = B(k; n, p_e) \cdot \sum_{k'} a_{k',n} \qquad (4)$$

and exclude (k, n) if $e_{k,n} > 0.01 \alpha_{k,n}$. More than 99% of the remaining $\alpha_{k,n}$ originate from the $p_c$ component, allowing to estimate $p_c$ using an EM algorithm that treats the excluded $X = \{(k_1, n_1), ...\}$ as missing data. If enough reads (we used 10,000 reads as threshold) remain, which was the case in all data sets but the 45 min labeling experiments, $p_c$ can be estimated with sufficient precision. Otherwise, the implementation may stop with an error.

Importantly, this will only happen when extremely few labeled RNA fragments are in the sample. The E step consists of replacing excluded read counts by their expected values given the current estimate $p_c^{(t)}$:

$$a_{k,n}^{(t+1)} = \frac{\sum_{(k',n) \notin X} B(k; n, p_c^{(t)}) \cdot a_{k',n}}{\sum_{(k',n) \notin X} B(k'; n, p_c^{(t)})} \qquad (5)$$

The M step computes a better estimate for $p_c$ as

$$p_c^{(t+1)} = \frac{\sum_{k,n} k a_{k,n}^{(t+1)}}{\sum_{k,n} n a_{k,n}^{(t+1)}} \qquad (6)$$

We noticed that running the EM algorithm led to extremely slow convergence rates. Thus, we use the following bisection scheme instead: For the search interval [l, r] (starting with 1 = 0 and r = 1), we set $p(t) = \frac{l+r}{2}$ and compute $p^{(t+1)}$ by a single EM iteration. If $p(t+1) < p(t)$, we continue with the search interval $[l, p^{(t)}]$, otherwise with $[p^{(t)}, r]$. We stop if $r - l < 10^{-8}$.

In principle, we compute the integral by dividing [0,1] into k equisized intervals and employ Newton-Cotes quadrature using the trapezoidal rule. This also gives straight-forward access to any credible interval. To allow for relatively small $k$ even for potentially extremely narrow posterior distributions $f$, we first identify the mode $m$ of $f$ by numerically maximizing

$$g(\pi_g; \mathbf{y}, \mathbf{n}, p_e, p_c) = \sum_i \log\left(P(y_i; p_e, p_c, n, \pi_g)\right) \cdot \log b(\pi_g; \alpha, \beta)$$

$$(7)$$

Then, we identify the values $l < m$, where $f(l) = 10^{-3}f(m)$ and $h > m$ where $f(h) = 10^{-3}f(m)$ by bisection. The interval $[l,h]$ contains most of the probability mass, so we use this interval for the numerical integration.

Finally, we noticed that the posterior distribution for any gene g closely resembles a beta distribution with density $b_g$. Having a closed-form representation for the posterior is important for subsequent steps. Therefore, we fit parameters $\alpha_g$ and $\beta_g$ by numerically minimizing the sum of squares computed between $f$ and $b_g$ for all Newton-Cotes points.

For the abundance $\alpha$ of an RNA with transcription rate $\sigma$ and decay rate $\delta$, the change over time is modeled by the following differential equation:

$$\frac{da}{dt} = \sigma - \delta a(t) \qquad (8)$$

With an initial abundance $\alpha_0$, this has the following closed-form solution:

$$a(t) = \left(a_0 - \frac{\sigma}{\delta}\right)e^{-t\delta} + \frac{\sigma}{\delta} \qquad (9)$$

Setting the initial abundance to zero for newly synthesized RNA and to the steady state for pre-existing RNA, we obtain the following functions for the abundance of new RNA $a_{new}$ and old RNA $a_{pre}$:

$$a_{new}(t) = -\frac{\sigma}{\delta}e^{-t\delta} + \frac{\sigma}{\delta} \qquad (10)$$

$$a_{pre}(t) = \frac{\sigma}{\delta}e^{-t\delta} \qquad (11)$$

Thus, at any time t, the proportion of new to old RNA is

$$\pi(t) = \frac{a_{new}(t)}{a_{new}(t) + a_{pre}(t)} = 1 - e^{-t\delta} \qquad (12)$$

This can be used to transform the decay rate into a proportion $\pi$ at time $t$ and vice-versa:

$$\delta_t(\pi) = -\frac{1}{t}\log(1 - \pi) \qquad (13)$$

$$\pi_t(\delta) = 1 - e^{-t\delta} \qquad (14)$$

Hence, for gene g if at any time $t$, the proportion of new and old RNA is an approximately beta distributed random variable $P(t) \sim Beta(\alpha, \beta)$ with density function $b_g(\pi, \alpha,\beta)$, the density function $d(\delta; \alpha,\beta)$ of the distribution of the transformed random variable $D^{(t)} = \delta_t(P^{(t)})$ can be found by substitution:

$$d(\delta; \alpha, \beta) = b_g\left(\pi_t(\delta); \alpha, \beta\right)\frac{d\pi_t}{d\delta} \qquad (15)$$

$$= \frac{t}{B(\alpha, \beta)} \left(1 - e^{-t\delta}\right)^{\alpha - 1} \cdot e^{-t\beta\delta} \qquad (16)$$

Thus, if several approximate posterior beta densities defined by $(\alpha_1, \beta_1),...,(\alpha_n, \beta_n)$ for proportion parameters measured at times $t_1, ..., t_n$ are given, the maximum a posteriori estimator for the decay rate $\delta$ can be found by numerically maximizing:

$$l(\delta) = \sum_i (\alpha_i - 1) \log(1 - e^{-t_i \delta}) - t_i \beta_i \delta \qquad (17)$$

Finally, the estimated decay rate $\delta$ can be transformed into an estimate of the RNA half-life $\lambda$ by:

$$\lambda = \frac{\log(2)}{\delta} \qquad (18)$$

We utilized available SLAM-seq data to determine realistic parameters for simulation. Specifically, we downloaded the processed table of a random sample (GSM2666852) from GEO and converted the CPM (read counts per million) into a read count distribution for genes by multiplying all CPM values by 20 million. Next, we downloaded the table containing half-lives estimated from their pulse-chase experiment and applied equations 18 and 14 to derive a realistic distribution of new to old proportions for a putative experiment with 3h 4sU labeling.

Above has been presented a statistical method to precisely estimate the proportion of new and old RNA in such experiments. This is based on a binomial mixture model, where the number of observed, experiment-specific mismatches is generated from one of two binomial distributions for reads from labeled and unlabeled RNA molecules. The output of our method is the full posterior distribution of the proportion of new and old RNA. This posterior is narrow for genes with many reads and for experiments with high incorporation and low sequencing error rates. Thus, it provides a straight-forward means for quality control.

In addition to sufficient incorporation rates that should be achieved, additional considerations for planning such experiments are as follows: Herzog et al. (2017) used single-end sequencing with read length 50bp on a Illumina HiSeq 2500. With the four-color chemistry, the HiSeq 2500 provides the smallest error rates possible today. Sequencing devices with two-color chemistry should preferably be avoided due to significantly greater error rates ($\sim$ 10x). Longer reads are generally preferable, as the probability of catching a modified nucleotide increases with longer reads.

FIG. 8 shows a validation of the method by simulation. (A) Estimation accuracy for the conversion rate $p_c$ is shown as the deviation of the estimated value from the true value in percentage of the true value. The error rate $p_e$ must be known to estimate $p_c$. Either the true error rate (known $p_e$) is supplied to the algorithm, or the true error rate plus a normally distributed error (according to parameters inferred from the no4sU experiments from Herzog et al. (2017), see FIG. 10C; estimated $p_e$) (B) 90% credible intervals and the posterior means for the proportion parameter $\pi$g are shown for 70 randomly sampled simulated genes. Here, the true $p_c$ and $p_e$ have been supplied to estimate. (C) Cumulative distributions of the absolute deviation from the true proportion are shown for all 18.917 simulated genes split by their read count n. Distributions for 100 simulations are overlayed. (D) The percentage of genes within equal-tailed credible intervals (CI; x axis) is shown for all 18.917 simulated genes split by their read count n. As in (C) 100 simulations are overlayed.

Data for FIG. 8 were simulated by the following procedure: We simulated as many genes as in the read count distribution. For each gene, we randomly sampled a read count from this distribution and a $\pi_g$ from the proportion distribution (except for FIG. 8B, where we set $\pi_d = 0.5$ for all genes). For each read, we first sampled the total number of thymines n from a binomial distribution with parameters L (read length, here L = 50 as in the available experiments) and u (thymine content, here we set u = 0.3 as computed from the 3' end investigated by Herzog et al. (2017)). Then, we determined whether this read originated from a new RNA (with probability $\pi_g$) or old RNA (with probability 1 - $\pi_g$). Finally, the number of T to C mismatches k was drawn from a binomial distribution with parameters n and either $p_e$ or $p_c$ (here we set $p_e = 1 \times 10^{-4}$ and $p_c = 0.023$, compare FIG. 10).

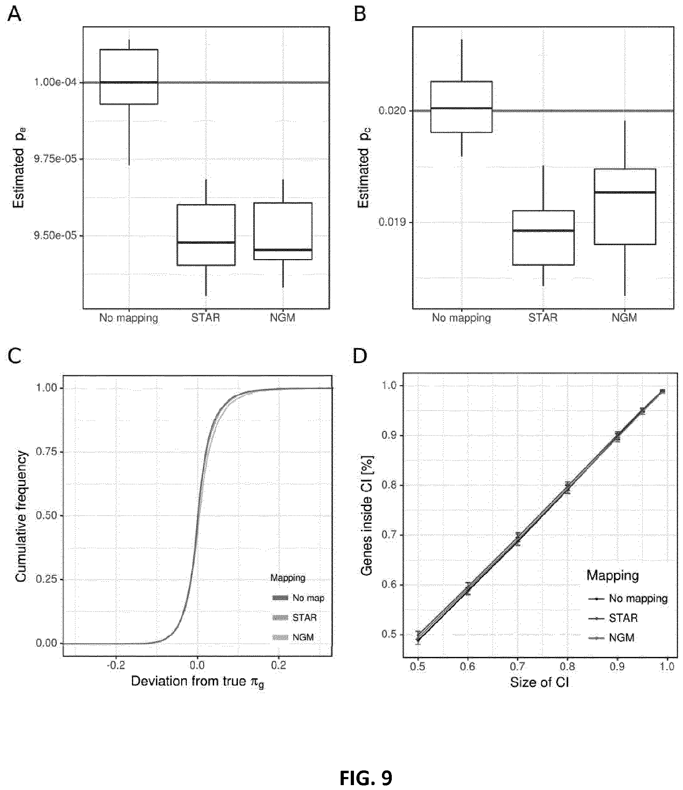

FIG. 9 shows the influence of read mapping. (A and B) We simulated ten data sets of reads and either used the true locations of the reads (No mapping) as input for the method, or a fastq file for STAR or NGM. Here, the distributions of the estimated error rates (A) and conversion rates (B) are shown. The true values are indicated. Read mapping with both STAR and NGM led to slightly, but significantly biased estimates. (C) The cumulative distribution of the absolute deviation from the true proportion is shown for reliably quantified genes (at least 100 reads). In spite of underestimated rates, read mapping effects on estimating the proportion are negligible. (D) The percentage of genes within equal-tailed credible intervals (CI; x axis) is shown. Read mapping does not affect the accuracy of credible intervals. Error bars indicate the standard deviation of the ten simulations.

Reads for FIG. 9 were generated in a similar manner, but here we directly selected a random read location from the gene 3' regions defined by Herzog et al. (2017) and generated mismatches accordingly (all twelve possible mismatches with rate $p_e$ or $p_c$ when appropriate). Here, a fixed $\pi_g = 0.2$ was used. Read locations were either directly written to a read mapping file, or sequences were generated and written to fastq files.

To map simulated reads or available SLAM-seq data we used STAR 2.5.3a (Dobin et al., 2013) with default parameters against a reference genome prepared from the murine genomic sequence and gene annotation from Ensembl version 90. We also mapped the simulated reads using NGM (Sedlazeck et al., 2013), which is utilized by SLAM-DUNK (Herzog et al., 2017) and can be parameterized specifically for SLAM-seq samples. For NGM we used the same parameters as used by SLAM-DUNK with the exception that we had to increase the gap penalty parameters since our method was not able to handle the format how Indels were reported by NGM. Of note, for the simulated data there were no true Indels. We handeled multimapping reads by fractional counts (e.g. if a read maps to three locations on the genome equally well, there is 1/3 of a read at each location).

Results

Metabolic labeling followed by RNA-seq in principle allows quantifying both pre-existing (i.e. before labeling) and newly transcribed RNA. In the SLAM-seq protocol (Herzog et al., 2017), RNA is labeled using 4-thiouridine, which is converted into a cytosin analog using iodoacetamide (IAA). Thus, libraries can readily be prepared for sequencing, and pre-existing and newly transcribed RNA can be distinguished based on observed T to C mismatches of reads mapped to the reference genome.

However, it is not possible to determine with certainty, whether an observed read originated from a newly transcribed or pre-existing RNA molecule: Sequencing errors produce T to C mismatches also on reads from old RNA, and because of relatively infrequent 4sU incorporations ($\sim 2\%$ of all uridines are replaced), a substantial fraction of reads from new RNA will not have a T to C mismatch. Of note, only a minority will contain more than one T to C mismatch. Nevertheless, based on all reads mapped to a gene, it is possible to statistically infer the proportion of new and old RNA.

To this end, we use a statistical model based on a binomial mixture model (see FIG. 7). We assume that the number of observed T to C mismatches for a read is generated by one of two binomial distributions. One corresponds to old RNA and its success probability parameter is the average T to C error rate. The other models new RNA and its parameter is the combined error and incorporation rate. Naturally, the mixture parameter of the model corresponds to the proportion of new and old RNA.

We are not only interested in computing a point estimate of the proportion, but we also compute the posterior distribution on this parameter. This is of great interest here, as the accuracy of the estimator greatly depends on the number of reads mapped to a gene, and the difference between the conversion and error rates. Thereby, the size of credible intervals provide a potent quality measure for SLAM-seq experiments.

The first step of our method is to estimate the conversion and error rate parameters $p_c$ and $p_e$. Both may vary between samples, but we assume them to be constant for all genes from a single sample. Therefore, we use all reads from a sample to estimate $p_e$ and $p_c$. Because both probabilities are relatively small, standard techniques for estimation on the binomial mixture model failed. However, if $p_e$ is known, it is possible to estimate $p_c$ by an EM algorithm (see Methods for details). Estimating $p_e$ is more problematic, as it depends on an accurate estimate for $\pi$ (the overall proportion of new and old RNA in the sample), which, in turn, depends on accurate estimates for $p_c$ and $p_e$. Again, standard techniques based on EM algorithms failed. However, in principle, $p_e$ can be experimentally determined by spiking-in unlabeled RNA before IAA treatment. Alternatively, $p_e$ can be measured in additional experiments without 4sU labeling (no4sU sample). The problem with the approach based on no4sU samples is that measurements vary between samples and an externally measured value may not be accurate enough for precisely estimating $p_c$ and $\pi g$ (the proportion of new and old RNA for gene g) for each gene g. However, we noticed that the twelve different error rates were highly correlated between samples. Thus, T to C error rates can be estimated from the other error rates, which are measured in SLAM-seq experiments (see Methods for details).

Thus, our first check was how accurately $p_c$ could be estimated if $p_e$ is known (e.g. measured by RNA spike-ins) or if $p_e$ is estimated using additional no4sU samples. To this end, we simulated a hundred data sets with realistic values of $p_e$ and $p_c$. Then, we either supplied the true $p_e$ for estimating $p_c$, or a slightly deviating $p_e$ (based on observed deviations in the no4sU data sets). The estimates of $p_c$ were highly accurate (less than 1% deviation; see FIG. 8A). Importantly, this was the case when the true $p_e$ was used and when a slightly deviating $p_e$ was used.

Next, we tested how well the individual gene proportions $\pi_g$ could be estimated when $p_c$ and $p_e$ are known. Estimates were not biased, and always within the expected bounds given by credible intervals (see FIG. 8B). Finally, we expanded our simulations on a realistic scenario, i.e. $p_c$ and $p_e$ were estimated for simulated data, and then the $\pi g$ were estimated based on $p_c$ and $p_e$. Again, estimates were not biased, and especially for genes with many reads, highly accurate (less than 0.05 absolute deviation; see FIG. 8C). Moreover, the number of genes within any credible interval exactly matched the expected number in all cases. This means that observed deviations are not due to errors in the process of estimation, but are because of insufficient data. Thus, computed credible intervals provide a potent mean to judge the quality of a data set and the estimates for all genes.

So far, we directly simulated numbers ($k_i$, $n_i$), i.e. $k_i$ T to C mismatches were observed for $n_i$ thymines in read $i$. Even if read mapping has high sensitivity and specificity in finding the right location for all reads, correct read mapping is crucial especially for reads with one or more T to C mismatches. In Herzog et al. (2017), the authors extended their own read mapping software NGM specifically for the purpose of mapping SLAM-seq reads. Therefore, by generating sequencing reads in-silico, we tested whether read mapping by a standard tool (STAR; Dobin et al. (2013)) or NGM affected our method.

First, we compared how well error and conversion rates could be estimated when read locations were directly written into read mapping files or mapped with STAR or NGM. Interestingly, read mapping resulted in significantly reduced estimates for both $p_e$ and $p_c$ (see FIG. 9A and B), indicating that indeed a substantial number of reads with simulated mismatches was either not mapped at all, mapped to more than one location or mapped to a wrong location. Of note, STAR and NGM read mappings were affected by this to a highly similar degree. However, this does neither introduce bias into estimating gene proportions $\pi g$ (see FIG. 9C), nor does it affect the size of credible intervals (see FIG. 9D). In summary, there is room for improving read mapping for SLAM-seq, but our method is robust enough to handle reads mapped even by widely used standard read mapping tools.

Herzog et al. (2017) conducted several SLAM-seq experiments on murine embryonic stem cells (mESCs) with different periods of labeling (45 minutes, 3 hours, 12 hours and 24 hours). We examined the conversion and error rates $p_c$ and $p_e$ estimated by the method for each of these experiments. For the 45min experiments, $p_c$ could not be estimated because too few reads had more than one T to C conversion. In such a case, our implementation prints a warning. Thus, we excluded these experiments from further analyses.

FIG. 10 shows an evaluation of mESC data. (A) For all SLAM-seq experiments from Herzog et al. (2017), the estimated conversion rate $p_c$ is compared to the intronic and exonic T to C mismatch rates. (B) Linear regression analysis of $p_c$ against the intronic T to C mismatch rate. Slopes (s) and p values are indicated. For all three regressions $r^2 > 0.99$. (C) The distribution of the error rate $p_e$ as measured in the 15 no4sU samples is compared to the estimated error rates in the 27 4sU samples (see (A)). (D) $p_e$ can be predicted by linear regression of the other error rates. In the no4sU samples, $p_e$ can be directly measured by counting T to C mismatches. This shows the results of a leave-one-out cross validation in the no4sU samples comparing the predictions (x axis) against the measured values (y axis).

We first compared the estimated conversion rate with the observed T to C mismatch rates from exonic and intronic reads for all samples (see FIG. 10A). Estimated conversion rates were spread around slightly above 0.02 in all cases, and were not correlated to the period of labeling. Especially for the 3h samples, both exonic and intronic mismatch rates were substantially lower than the estimated conversion rates and were correlated to the period of labeling. For exons, this was expected since a substantial fraction of the total mature RNA is older than 3h. Interestingly, albeit to a lesser extent, we also observed this for intronic RNA, which is believed to be quickly degraded after splicing. The fact that the T to C mismatch rate is significantly higher after 12h of labeling than after 3h of labeling is indicative for frequent intron retention, or that at least some introns are relatively long-lived.

Intronic RNA was excluded from estimating conversion rates, but there was nevertheless a high correlation ($r^2 > 0.99$) of intronic T to C mismatch rates with estimated conversion rates. This indicates that conversion rates were estimated very accurately, and that a certain amount of intronic RNA is older than 3, 12 or 24 hours. Regression analysis revealed these amounts to be 70%, 91% and 92% in mESCSs, respectively (see FIG. 10B).

In Herzog et al. (2017), 15 samples have also been measured without 4sU labeling. For these all RNA is by definition old, and the mixture model reduces to a model with a single binomial component. Thus, $p_e$ is directly measured in these samples. Interestingly, the measured $p_e$ varied between $0.8 \times 10^{-3}$ and $1.6 \times 10^{-3}$ (see FIG. 10C). Thus, taking such a measured $p_e$ for another sample where the sample specific $p_e$ is some value within this range can lead to biased estimates of the new to old proportions $\pi_g$. This can be circumvented by either directly measuring $p_e$ in each sample using RNA spike-ins, or by employing a linear regression based estimation of $p_e$: We noticed that between the no4sU samples other error rates (e.g. T to A) were highly correlated to T to C error rates. Thus, we trained a linear regression model in the no4sU samples to estimate T to C error rates. Of note, estimated T to C error rates from the samples with 4sU were in the same range as observed error rates in the no4sU samples (see FIG. 10C), and the T to C error rates in the no4sU samples could be predicted with high accuracy (see FIG. 10D).

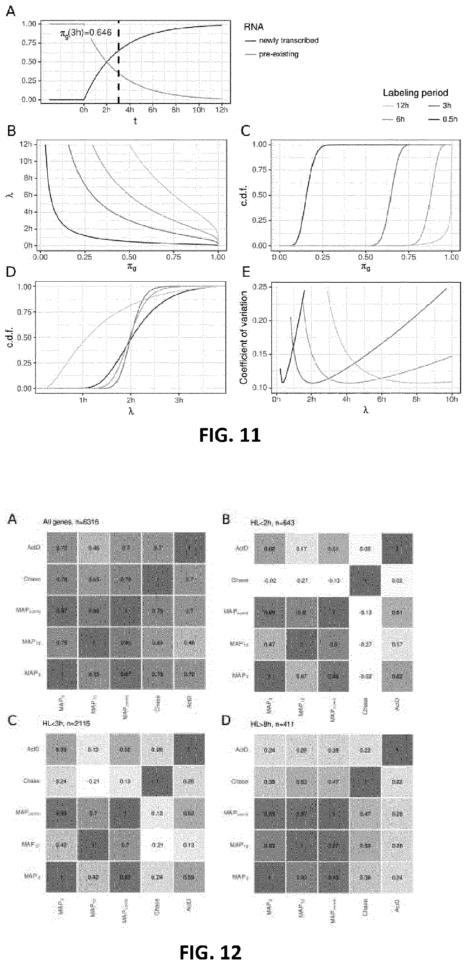

FIG. 11 illustrates RNA half-life (A) The proportion of new and old RNA of a gene g at any time t, $\pi_g(t)$, is directly related to its RNA half-life (here, 2h). (B) The functions are shown that transform the proportion $\pi g$ to the RNA half-life for different periods of labeling (see common legend of subfigures B to E at top right corner). (C) Posterior distributions of a theoretical gene g with 1000 reads and an RNA half-life of 2h for the four different periods of labeling. (D) These posterior distributions for $\pi g$ translate to specific posterior distributions on $\lambda$, with the one for t = 3h being the most precise one. (E) Coefficients of variation (standard deviation divided by the mean) of the posterior distributions on $\lambda$ for theoretical

genes with RNA half-lives between 0 and 10h.

The proportion $\pi_g$ of new and old RNA after some period of labeling t can be transformed into the RNA half-life $\lambda_g$ (see FIG. 11A). The functions $f_t$ transforming $\pi_g$ into $\lambda_g$ vary greatly for different values of t. Naturally, very short labeling periods (e.g. t = 1/2h) can resolve short RNA half-lives (e.g. $\lambda_g$ < 1h) very accurately, but small differences in $\pi_g$ result in large deviations of $\lambda_g$ for genes with long half-life (see FIG. 11B).

To analyze the variance in estimating RNA half-lives using our method, we first theoretically considered an experiment with typical parameters as observed in the data sets of Herzog et al. (2017), and a gene g with 1000 reads with a half-life of $\lambda_g$ = 2h. For different labeling periods, this gives rise to specific posterior distributions on the proportion parameter $\pi_g$ (see FIG. 11C) which can be transformed into posterior distributions on the estimated RNA half-life (see FIG. 11D). Interestingly, the estimate due to 3h labeling is the most precise, followed by 6h, 0.5h and 12h. We expanded this analysis to genes with different RNA half-lives, and computed the coefficient of variation (CV) of the posterior distribution of the estimated half-lives (see FIG. 11E). The CV is the standard deviation divided by the mean and therefore describes the expected relative deviation. The CV varied greatly depending on the labeling period, with short labeling periods generally most precise for genes with short RNA half-life. In addition, each labeling period has a range of true RNA half-lives where it is most precise and it extremely imprecise for too long or short-lived genes. E.g. with 3h labeling, estimation precision deteriorates for genes with a half-life below half an hour or longer than 8h. Thus, to precisely estimate the whole range of RNA half-lives in an experiment, several samples with different labeling periods are necessary as well as a method that automatically weighs the contributions of each sample to the overall estimate based on the varying variances. This can be achieved by maximum a posteriori (MAP) estimation of the RNA decay rate.

Herzog et al. (2017) estimated RNA half-lives by pulse-chase experiments: To achieve sufficient labeling, cells were supplied with 4sU for 24h. After that 4sU was washed out and the drop of conversions was monitored for several time points via SLAM-seq. RNA half-lives were then estimated by fitting an exponential decay model using least squares. These experiments are relatively laborious and introduce the wash-out efficiency as an additional source for potential bias. Furthermore, the least squares fitting does not respect the varying precision of estimating different half-lives with different labeling periods. For comparison, RNA half-lives were also determined using actinomycin D (ActD) treatment and monitoring the drop of RNA levels over time using RNA-seq.

FIG. 12 illustrates Pearson's correlation coefficient for RNA half-lives (A) For n = 6.316 genes, the correlation between any pair of methods estimating RNA half-lives was computed. $MAP_3$, MAP12 and $MAP_{comb}$ are the maximum a posterior estimators of the method computed on the 3h, 12h samples or both. Chase is the exponential decay model fit of Herzog et al. (2017) on the pulse-chase experiments. ActD is the exponential decay model fit for the actinomycin D experiment. (B-D) Correlation coefficients for different subsets of genes split according to ultra-short RNA half-life, short RNA half-life and long RNA half-life.

In addition to the pulse-chase and ActD estimates, we used the 3h or 12h labeling data or their combination to estimate RNA half-lives for mESCs using our maximum a posteriori approach ($MAP_3$, $MAP_{12}$, $MAP_{comb}$). The correlation coefficients computed over all genes also utilized for comparison in Herzog et al. (2017) showed that $MAP_{comb}$ performed equally well (R $\approx$ 0.7) as the pulse-chase experiments in reproducing the ActD estimates (see FIG. 12A). $MAP_3$ resulted in a similarly high correlation but the $MAP_{12}$ estimates showed worse correlation (R $\approx$ 0.46). For genes with ultra-short (< 2h) and short (< 3h) RNA half-lives however, the correlation of the pulse-chase experiment was poor (R $\approx$ 0 and R $\approx$ 0.26, respectively), and significantly better for $MAP_3$ and $MAP_{comb}$ (R > 0.59 and R > 0.49). For genes with longer RNA half-lives, correlations were generally poor, but $MAP_{12}$ provided the highest correlations (see FIG. 12D).

Furthermore, $MAP_{comb}$ always resulted in correlation coefficients (computed for the comparison to the pulse-chase or the ActD experiment) that were close to the better of $MAP_3$ or $MAP_{12}$. This indicates that the MAP estimation of the RNA half-live effectively weighs the different precisions obtained for measuring with different labeling periods.

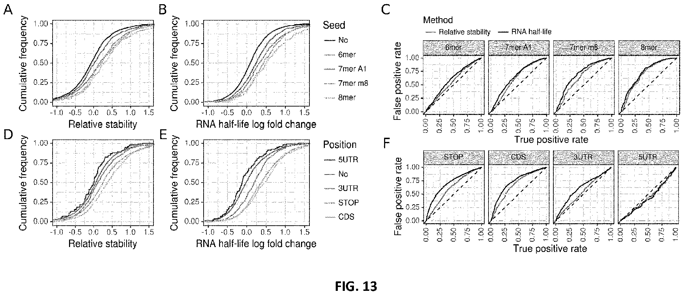

FIG. 13 provides a differential analysis (A-B) We repeated the microRNA target prediction analysis of Herzog et al. (2017). Relative stability values (A) are computed from the corrected conversion counts from the 3h and 12h experiments, RNA half-life log2 fold changes using the method (B). The RNA half-lives show a stronger enrichment of targets upon Xpo5 knock-out for all seed types. (C) We performed ROC analyses by treating predicted microRNA targets as the true objects, and mRNAs without seed as the false objects. Then, either the relative stability or RNA half-life log2 fold change was taken as prediction score. MicroRNA target predictions agreed better with RNA half-lives than with relative stabilities for all four seed types. (D-F) We repeated the m6A modification analyses from Herzog et al. (2017). As expected, no enrichment upon Mettl$_3$ knock-out was found for mRNAs with m6A in the 5' UTR. For all other mRNA locations defined in Batista et al. (2014), the RNA half-lives show a substantially stronger enrichment of m6A containing mRNAs.

Several factors are known to affect the stability of specific RNAs. Most prominently, microRNAs are small RNAs expressed by virtually all eukaryotic cells, that (imperfectly) basepair to cognate sites in mRNAs. Thereby, they guide the RNA induced silencing complex (RISC) to target mRNAs, which leads to translational repression and induces RNA decay (Bartel, 2009; Jonas and Izaurralde, 2015). By knocking-out Exportin-5 (Xpo5), an essential factor in the biogenesis of the most abundant family of microRNAs in mESCs (miR-291a), repression of mRNA targets of this family is reduced, effectively prolongating their RNA half-life.

In Herzog et al. (2017), this has been analyzed by considering the relative RNA stability computed by comparing the (no4sU corrected) T to C mismatch rates for wild-type (wt) and Xpo5 knock-out (ko) cells. This indeed revealed different sets of predicted microRNA targets to have increased RNA stabilities (see FIG. 13A). Using the presented method we were able to compute RNA half-lives for both conditions (wt and ko) and compute their log2 fold changes (see FIG. 13B). log2 fold changes already indicates that the latter is a better measure to capture the action of the microRNAs. Indeed, receiver operating characteristic (ROC) analysis revealed RNA half-life log2 fold changes to better agree with predicted microRNA targets than relative RNA stability. However, the overall difference between genes predicted to be a microRNA target and the remaining genes is generally poor, presumably due to secondary effects of knocking down Xpo5 or the difficulty in predicting microRNA targets.

Another cellular mechanism affecting RNA stability is N6 adenosine methylation (m6A) (Meyer and Jaffrey, 2014; Yue et al., 2015). It has been shown that m6A at specific mRNA locations induces mRNA degradation. m6A modification is performed by the protein complex N6-adenosine-methyltransferase. Thus, by knocking out its 70 kDa subunit (Mettl$_3$), genes affected by m6A mediated degradation that have been experimentally determined in mESCs are de-repressed. Similarly to the microRNA analyses, relative RNA stabilities can reveal this (see FIG. 13A), but RNA half-lives computed by the presented method reveal substantially more differences between targets and non-targets (see FIG. 13B and C).

The estimation of sample specific error rates is an important component of our method. Here, this has been solved by the observation that error rates were correlated, which we could exploit by a linear regression model. The model was trained on available samples that have not been treated with 4sU, and could predict T to C error rates with sufficient accuracy. A potent alternative would be to use RNA spike-ins such as the ERCC mix (Jiang et al., 2011) in each sample. This way, error rates could be directly estimated by counting mismatches on the ERCC RNAs. We have shown that the estimated proportions of new and old RNA can be used to compute precise RNA half-lives. This (and all other estimates of RNA half-life based on metabolic labeling) heavily relies on the incorporation rate of the nucleoside analog to be constant over time. Considering that they have to cross cell membranes, the cytoplasm and the nuclear membrane to increase their concentration in the nucleus, we expect this assumption to be problematic. 4sU needs time to accumulate, and methods are needed to measure this effectively reduced time of labeling to be considered in estimating RNA half-lives. We uncovered that using SLAM-seq, short half-lives can be resolved more precisely with short periods of labeling.

**Claims**

1. A method for determining a quantification of old and new RNA for a gene, the method comprising:

    - obtaining reads from RNA fragments of a sample, wherein the RNA fragments comprise nucleotide substitutions caused by a metabolic marker,
    - determining (130) statistics of nucleotide mismatches in the reads,
    - determining (140, 150), based on the statistics of nucleotide mismatches, an error rate indicating a rate of nucleotide mismatches in old RNA and a conversion rate indicating a rate of nucleotide mismatches in new RNA, and
    - determining (160) the quantification of old and newly synthesized RNA for the gene based on the error rate, the conversion rate and the statistics for the gene.

2. The method of claim 1, wherein the metabolic marker is 4sU and/or wherein the nucleotide mismatches are T to C mismatches.

3. The method of one of the previous claims, further comprising a preprocessing step of removing from the statistics nucleotide substitutions which, due to their high number, cannot be explained as conversions by the metabolic marker.

4. The method of one of the previous claims, wherein determining the error rate comprises using a linear regression model that has been trained with reads of cells that have not been metabolically labelled to predict the error rate based on error rates of mismatches that do not correspond to a metabolically caused mismatch.

5. The method of one of the previous claims, wherein determining the conversion rate comprises:

    - determining a number k of nucleotide mismatches for which less than a predetermined ratio of observed reads is expected to originate from RNA that is not metabolically labelled, and

- determining the conversion rate based on statistics where k or fewer mismatches are excluded.

6. The method of one of the previous claims, wherein the reads are acquired using paired-end-sequencing and wherein the method further comprises determining a double-stranded error rate indicating a rate of nucleotide mismatches in double-sequenced parts of read pairs of old RNA and/or a double-stranded conversion rate indicating a rate of nucleotide mismatches in double-sequenced parts of read pairs of new RNA.

7. The method of one of the previous claims, further comprising determining (170) the RNA half-life $\lambda$ of the gene according to

$$\lambda = \frac{\log(2)}{-\frac{1}{t}\log(1-\pi)}$$

wherein t is the effective labeling time of the metabolic marker, in particular of 4sU, and $\pi$ is the ratio of new to total RNA.

8. The method of one of the previous claims, further comprising:

- determining a probability distribution of a ratio of new-to-total RNA for a plurality of samples,
- for each of the plurality of samples, determining parameters of a beta distribution that approximates the probability distribution, and
- determining an estimator of the decay rate or a posterior distribution of the estimator of the decay rate based on the parameters of the plurality of plurality of samples, and/or
- determining an estimator of the RNA half-life or a posterior distribution of the estimator of the RNA half-life based on the parameters of the plurality of plurality of samples.

9. The method of claim 8, wherein determining parameters of a beta distribution that approximate the probability distribution comprises fitting parameters of a beta distribution by numerically minimizing a distance measure between the probability distribution and the beta distribution.

10. The method of claim 8 or 9, wherein the estimator is determined by numerically maximizing:

$$l(\delta) = \sum_i (\alpha_i - 1)\log(1 - e^{-t_i\delta}) - t_i\beta_i\delta$$

wherein $\delta$ is the decay rate, $\alpha_i$, $\beta_i$ are parameters of an i-th sample, and $t_i$ is a labeling time of the metabolic marker in the i-th sample.

11. The method of one of the previous claims, further comprising: determining, based on the probability distribution of the ratio of newly synthesized RNA of the gene and/or based on the probability distribution of the RNA half-life of the gene, a biological significance of the sample.

12. The method of claim 11, further comprising rejecting or accepting a determined value of the sample based on the determined biological significance.

13. The method of one of the previous claims, wherein old RNA is labeled by the metabolic marker.

14. A device for determining a quantification of old and new RNA for a gene, the device comprising:

- an obtaining unit for obtaining reads from RNA fragments of a sample, wherein the RNA fragments comprise nucleotide substitutions caused by a metabolic marker,
- a statistics unit for determining (130) statistics of nucleotide mismatches in the reads,
- a sample analysis unit for determining (140, 150), based on the statistics of nucleotide mismatches, an error rate indicating a rate of nucleotide mismatches in old RNA and a conversion rate indicating a rate of nucleotide mismatches in new RNA, and
- a gene analysis unit for determining (160) the quantification of old and newly synthesized RNA for the gene based on the error rate, the conversion rate and the statistics for the gene.

15. A computer-readable storage medium storing program code, the program code comprising instructions that when executed by a processor carry out the method of one of claims 1 to 13.

**FIG. 1**

#T to C    n=10

| | |
|---|---|
| 0 | 91897 |
| 1 | 6400 |
| 2 | 1492 |
| 3 | 198 |
| 4 | 13 |

**FIG. 2**

**FIG. 3**

# T to C   n=10, old   n=10, new

| | | |
|---|---|---|
| 0 | 80020 | 11877 |
| 1 | 76 | 6324 |
| 2 | 0 | 1492 |
| 3 | 0 | 198 |
| 4 | 0 | 13 |

**FIG. 4**

**FIG. 5**

**FIG. 6**

**FIG. 7**

**FIG. 8**

**FIG. 9**

FIG. 10

**FIG. 11**

**FIG. 12**

**FIG. 13**

Europäisches
Patentamt

European
Patent Office

Office européen
des brevets

## EUROPEAN SEARCH REPORT

Application Number

EP 18 17 9371

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HERZOG VERONIKA A ET AL: "Thiol-linked alkylation of RNA to assess expression dynamics", NATURE METHODS, NATURE PUBLISHING GROUP, GB, 25 September 2017 (2017-09-25), XP009500433, ISSN: 1548-7105, DOI: 10.1038/NMETH.4435 * abstract * * page 1199, left-hand column, last paragraph - right-hand column, paragraph 1 * * Section: Online Methods * | 1-15 | INV. C12Q1/68 G06F19/18 C12Q1/6869 G06F19/22 G06F19/12 |
| X | -& VERONIKA A HERZOG ET AL: "Thiol-linked alkylation of RNA to assess expression dynamics", NATURE METHODS, vol. 14, no. 12, 25 September 2017 (2017-09-25), pages 1198-1204, XP055502021, New York ISSN: 1548-7091, DOI: 10.1038/nmeth.4435 * figure 8 * ----- | 1-15 | |
| X | Marisa Baptista ET AL: "RNA dynamics revealed by metabolic RNA labeling and biochemical nucleoside conversions", Nature methods, 28 February 2018 (2018-02-28), pages 171-172, XP055500889, United States DOI: 10.1038/nmeth.4608 Retrieved from the Internet: URL:https://www.nature.com/articles/nmeth.4608.pdf [retrieved on 2018-08-24] * figure 1 * * the whole document * ----- -/-- | 1-15 | TECHNICAL FIELDS SEARCHED (IPC) C12Q G06F |

The present search report has been drawn up for all claims

| Place of search | Date of completion of the search | Examiner |
|---|---|---|
| Munich | 3 September 2018 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BERND RÄDLE ET AL: "Metabolic Labeling of Newly Transcribed RNA for High Resolution Gene Expression Profiling of RNA Synthesis, Processing and Decay in Cell Culture", JOURNAL OF VISUALIZED EXPERIMENTS, no. 78, 8 August 2013 (2013-08-08), XP055501988, DOI: 10.3791/50195 * page 8, paragraph 7 - page 10, paragraph 1 * | 1-15 | |
| X | Wayo Matsushima ET AL: "SLAM-ITseq: Sequencing cell type-specific transcriptomes without cell sorting", bioRxiv, 16 December 2017 (2017-12-16), XP055502086, DOI: 10.1101/235093 Retrieved from the Internet: URL:https://www.biorxiv.org/content/biorxiv/early/2017/12/16/235093.full.pdf [retrieved on 2018-08-24] * page 5, line 3 - line 6 * * page 10, last paragraph * * page 11, last paragraph - page 12, last paragraph * | 1-15 | |
| A | C. MILLER ET AL: "Dynamic transcriptome analysis measures rates of mRNA synthesis and decay in yeast", MOLECULAR SYSTEMS BIOLOGY, vol. 7, no. 1, 16 April 2014 (2014-04-16), pages 458-458, XP055500892, DOI: 10.1038/msb.2010.112 * the whole document * | 1-15 | |

TECHNICAL FIELDS SEARCHED (IPC)

-/--

The present search report has been drawn up for all claims

| Place of search | Date of completion of the search | Examiner |
|---|---|---|
| Munich | 3 September 2018 | Ulbrecht, Matthias |

Europäisches
Patentamt

European
Patent Office

Office européen
des brevets

## EUROPEAN SEARCH REPORT

Application Number

EP 18 17 9371

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CAROLINE C. FRIEDEL ET AL: "Conserved principles of mammalian transcriptional regulation revealed by RNA half-life", NUCLEIC ACIDS RESEARCH, vol. 37, no. 17, 26 June 2009 (2009-06-26), pages e115-e115, XP055500894, ISSN: 0305-1048, DOI: 10.1093/nar/gkp542 * the whole document * ----- | 1-15 | |
| A | ALEXANDER DOBIN ET AL: "STAR: ultrafast universal RNA-seq aligner", BIOINFORMATICS., vol. 29, no. 1, 25 October 2012 (2012-10-25), pages 15-21, XP055500895, GB ISSN: 1367-4803, DOI: 10.1093/bioinformatics/bts635 * the whole document * ----- | 1-15 | |

TECHNICAL FIELDS
SEARCHED      (IPC)

The present search report has been drawn up for all claims

| Place of search | Date of completion of the search | Examiner |
|---|---|---|
| Munich | 3 September 2018 | Ulbrecht, Matthias |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)